# EUROPEAN PATENT APPLICATION

(11) **EP 2 905 046 A1**
(43) Date of publication of application: **12.08.2015**
(21) Application number: 12885994.9
(22) Date of filing: 04.10.2012
(51) Int. Cl.: A61M 25/00

(54) **STYLET FOR MEDICAL NEEDLE AND MEDICAL NEEDLE**

(71) Applicant: Unisis Corp., Tokyo 110-0016 (JP)
(72) Inventor: SAITOH, Hideya, Tokyo 110-0016 (JP); YAMAZAKI, Toru, Koshigaya-shi Saitama 343-0822 (JP)
(74) Representative: Brown, David Leslie
(86) International application number: PCT/JP2012/075817
(87) International publication number: WO 2014/054156

(57) **Abstract**

A stylet can be easily removed from a needle main body even when a needle section is bent, and contributes to an advanced medical treatment such as percutaneous laser disk decompression (PLDD). The stylet includes a rod-like section, and a base end block that is provided at a base end of the rod-like section, the base end block being engaged with a base end block of a needle main body for positioning, the rod-like section being inserted into the needle main body from a base end of the needle main body to close a front end opening of the needle main body, the entirety or part of the rod-like section being a helical coil formed by helically winding a wire. Since the helical coil is flexible, and decreases in diameter when pulled, friction with the inner surface of the needle section decreases, and the stylet can be easily removed from the needle main body even when the needle section of the needle main body is bent.

## Description

### TECHNICAL FIELD

The present invention relates to a stylet that is inserted into a medical needle that is for example used to introduce a catheter or a laser fiber into a body, to close the front end opening of the needle, and a medical needle that includes the stylet.

### BACKGROUND ART

Human tissue such as skin may be damaged when a medical needle having a large front end opening is inserted into a body. Therefore, a needle having a relatively large front end opening is inserted in a state in which a thin rod-like stylet is inserted into the needle to close the front end opening.

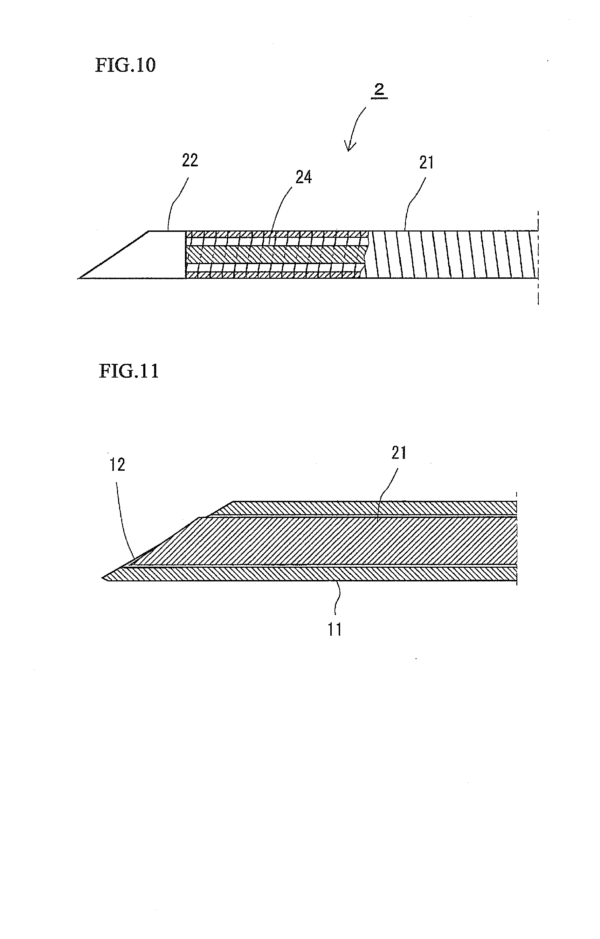

As illustrated in FIG. 11, a related-art stylet is formed in the shape of a simple rod (see Patent Literatures 1 and 2).

### CITATION LIST

### PATENT LITERATURE

PTL 1: JP-A-10-328302
PTL 2: Japanese Utility Model Registration No. 3152220

### SUMMARY OF INVENTION

### TECHNICAL PROBLEM

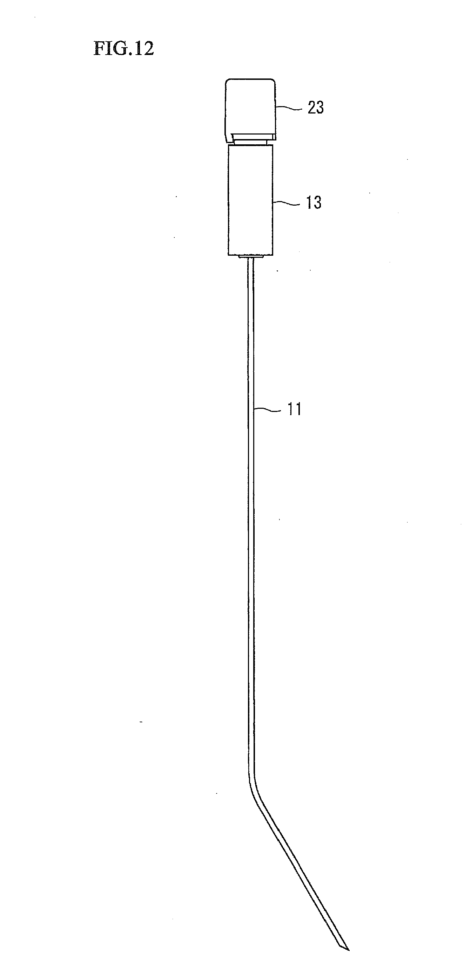

A doctor may insert a medical needle in a state in which the needle section is bent (see FIG. 12) when performing an advanced medical treatment developed in recent years. For example, percutaneous laser disk decompression (PLDD) is a medical treatment in which a needle is inserted into skin, the tip of a thin laser fiber is introduced into an intervertebral disk through the needle, the intervertebral disk is cauterized by applying a laser beam to form a cavity, and a hernial protrusion is returned to the original position. In this case, it may be necessary to bend the needle section in order to introduce the medical needle between the desired vertebrae. Specifically, the medical needle is inserted in a state in which the needle section is bent as illustrated in FIG. 12. After removing the stylet from the needle main body, the laser fiber is inserted into the needle main body, and the laser treatment is performed.

However, it is very difficult to remove the stylet from the needle main body when the needle section is bent, and it may be impossible to remove the stylet from the needle main body. In such a case, it may be necessary to remove the medical needle from the intervertebral disk, and insert the medical needle again after adjusting the degree of bending of the needle section.

An object of the invention is to make it possible to easily remove the stylet from the needle main body even when the needle section is bent, and contribute to an advanced medical treatment such as PLDD.

### SOLUTION TO PROBLEM

According to one aspect of the invention, a stylet for a medical needle includes a rod-like section, and a base end block that is provided at a base end of the rod-like section, the base end block being engaged with a base end block of a needle main body for positioning, the rod-like section being inserted into the needle main body from a base end of the needle main body to close a front end opening of the needle main body, the entirety or part of the rod-like section being a helical coil formed by helically winding a wire (claim 1).

The helical coil may be formed by helically winding a thin piano wire, for example. It is preferable that the helical coil has a circular or quadrangular cross-sectional shape, and have a diameter of about 0.1 to about 0.25 mm.

The entirety or part of the rod-like section may be the helical coil. It is preferable that the entirety of the rod-like section is the helical coil. When part of the rod-like section is the helical coil, the base end or the front end of the rod-like section may be formed of a known rod-like part, and the remaining part of the rod-like section may be the helical coil. The rod-like part and the helical coil may be connected by welding or the like.

Since the helical coil is flexible, and decreases in diameter when pulled, friction with the inner surface of the needle section decreases, and the stylet can be easily removed from the needle main body even when the needle section of the needle main body is bent.

The stylet for a medical needle may include a core that is provided inside the helical coil (claim 2).

A metal wire (e.g., stainless steel wire), a plastic wire, or the like may be used as the core. The core may have a thickness of about 0.2 to about 0.6 mm, for example. The rigidity of the rod-like section including the helical coil can be improved by providing the core. The rigidity of the rod-like section can also be adjusted by changing the thickness or the material of the core.

In the stylet for a medical needle, a front end of the rod-like section may have a structure in which a massive front end section is integrally provided to a front end of the helical coil (claim 3).

The massive front end section can be integrally provided to the front end of the helical coil by heating and melting the front end of the helical coil by an arc discharge or the like, and allowing the molten helical coil to solidify. The massive front end section may be separately provided, and welded to the front end of the helical coil.

In the stylet for a medical needle, the massive front end section may have a slope that is formed by diagonally cutting the front end section (claim 4).

The front end opening of the needle section is occupied to a large extent by the front end section of the stylet by forming the slope, and human tissue is damaged to only a small extent when inserting the medical needle.

According to another aspect of the invention, a medical needle includes the stylet according to any one of claims 1 to 4; and a needle main body that includes a needle section, and a base end block that is provided at a base end of the needle section and engaged with a base end block of the stylet (claim 5).

The stylet according to one aspect of the invention can be used in combination with a known needle main body.

According to another aspect of the invention, a medical needle includes the stylet according to claim 4; and a needle main body that includes a needle section, and a base end block that is provided at a base end of the needle section and engaged with a base end block of the stylet, the slope entirely forming a cutting face that protrudes from the front end opening of the needle main body, and a front end face of the needle main body not forming a cutting face (claim 6).

The medical needle is characterized in that the stylet has a cutting face, and the front end of the needle main body does not have a cutting face. Since the needle main body does not have a cutting face, a situation in which human tissue is damaged by the tip of the needle can be suppressed when performing a medical treatment in which a catheter or a laser fiber is inserted after removing the stylet.

Note that the term "cutting face" used herein refers to a front end face that forms an angle theta of 45° or less with the axis of the stylet or the needle main body.

### ADVANTAGEOUS EFFECTS OF INVENTION

The stylet and the medical needle according to the aspects of the invention can be advantageously used for a treatment that is performed in a state in which the needle section is bent since the stylet can be easily removed from the needle main body.

### BRIEF DESCRIPTION OF DRAWINGS

FIG. 1 is a side view illustrating a stylet and a needle main body according to one embodiment of the invention.
FIG. 2 is an enlarged view illustrating the front end of a stylet according to one embodiment of the invention.
FIG. 3 is a cross-sectional view illustrating the main part of a medical needle according to one embodiment of the invention.
FIG. 4 illustrates the front end of a stylet according to one embodiment of the invention.
FIG. 5 is a cross-sectional view illustrating the main part of a medical needle according to one embodiment of the invention.
FIG. 6 illustrates the front end of a stylet according to one embodiment of the invention.
FIG. 7 is a cross-sectional view illustrating the main part of a medical needle according to one embodiment of the invention.
FIG. 8 illustrates the front end of a stylet according to one embodiment of the invention.
FIG. 9 is a cross-sectional view illustrating the main part of a medical needle according to one embodiment of the invention.
FIG. 10 is a cutaway cross-sectional view illustrating the front end of a stylet according to one embodiment of the invention.
FIG. 11 is a cross-sectional view illustrating the main part of a related-art medical needle.
FIG. 12 illustrates a usage state of a medical needle.

### DESCRIPTION OF EMBODIMENTS

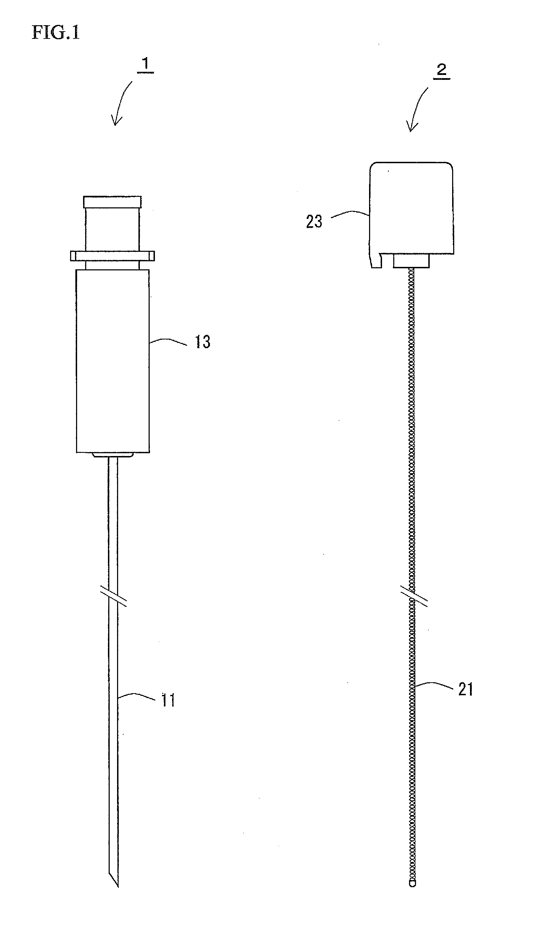

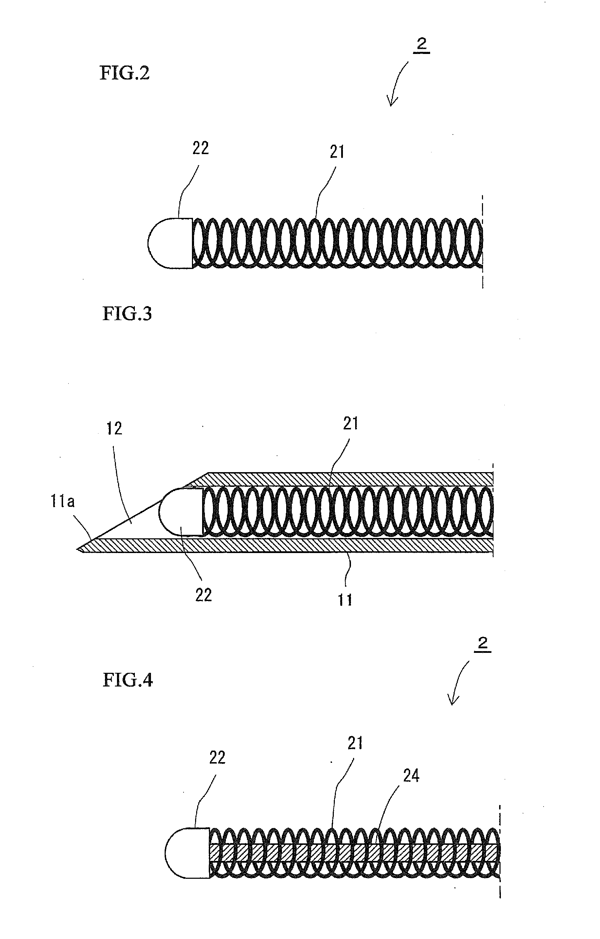

A stylet 2 illustrated in FIGS. 1 to 3 includes a base end block 23 that is made of a plastic, and a rod-like section 21 that protrudes forward from the center of the front surface of the base end block 23. The base end block 23 has the same structure as that of the base end block of a known stylet. The rod-like section 21 includes a helical coil that is formed by helically winding a thin piano wire, and a massive front end section 22 that is formed at the front end of the rod-like section 21 (see FIG. 2). The front end section 22 is formed by heating and melting the front end of the helical coil by an arc discharge, and allowing the molten helical coil to solidify through natural cooling.

A medical needle according to one embodiment of the invention is produced by combining the stylet 2 with a needle main body 1 illustrated in FIG. 1. The needle main body 1 has a configuration in which a base end block 22 that is made of a plastic and has an approximately tubular shape is secured on the base end of a needle section 21. The needle main body 1 has the same structure as that of a known needle main body. The needle section 21 has an outer diameter of 1.45 mm and an inner diameter of 1.1 mm, for example. When the rod-like section 21 of the stylet 2 is inserted into the needle main body 1 from the rear end of the base end block 13 of the needle main body 1, the base end block 23 of the stylet is engaged with the base end block 13 of the needle main body for positioning. In this case, the tip of the medical needle has the configuration illustrated in FIG. 3 in which a front end opening 12 of the needle section 11 is closed by the front end section 22 of the stylet. The front end face of the needle section 11 forms a cutting face 11 a that forms an angle of 45° or less with the axis of the needle section 11.

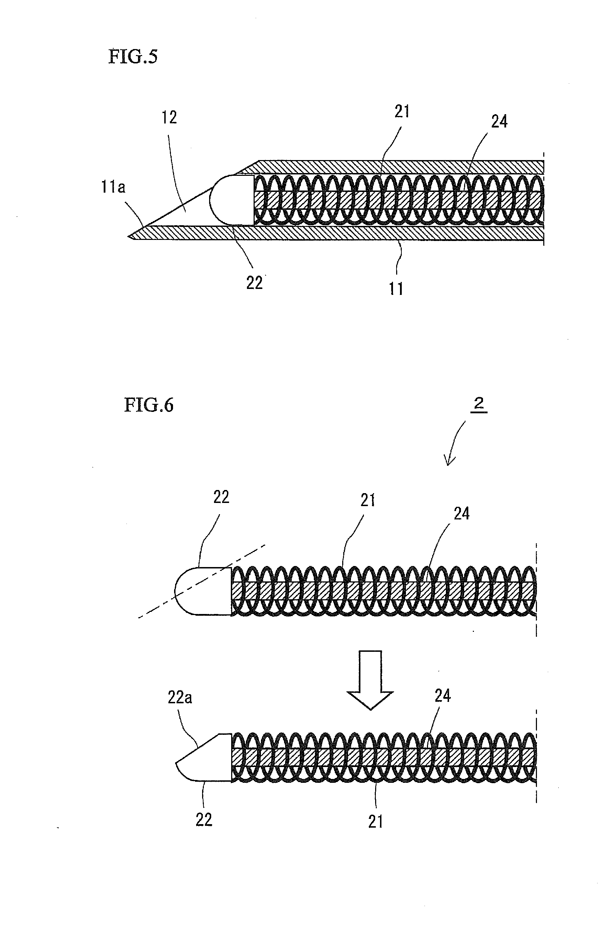

The stylet illustrated in FIGS. 4 and 5 has a configuration in which a core is provided inside the helical coil of the rod-like section 21 of the stylet illustrated in FIG. 2. The core is formed of a stainless steel wire. The front end of the core is welded to the front end section 22, and the base end of the core is secured on the base end block 23.

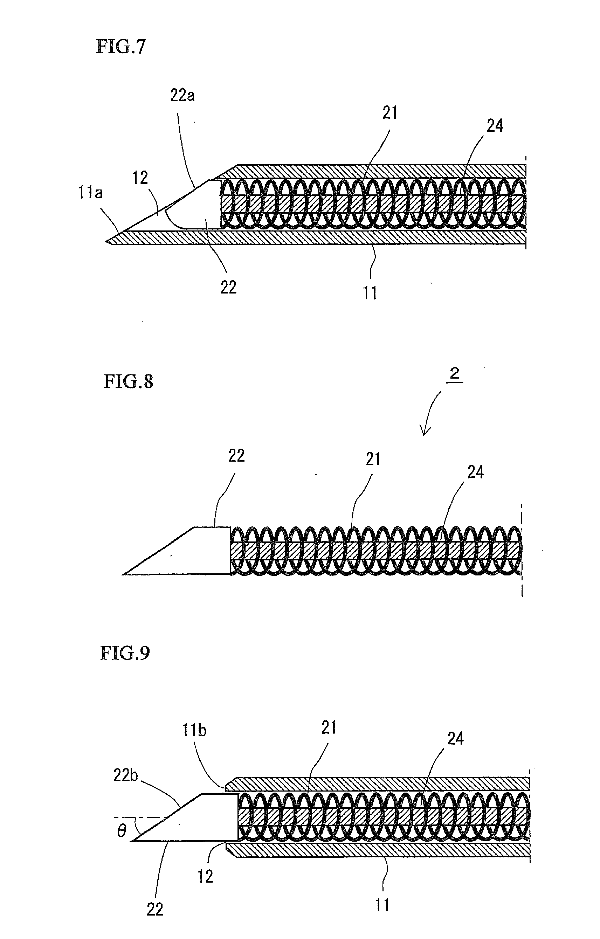

The stylet illustrated in FIGS. 6 and 7 has a configuration in which the front end section 22 of the stylet illustrated in FIG. 4 is diagonally cut to form a slope 22a. FIG. 5 illustrates the tip of a medical needle in which the stylet is inserted into a needle main body. The front end opening 12 of the needle section is occupied to a large extent by the front end section 22 of the stylet as compared with the case illustrated in FIG. 3 by forming the slope 22a, and human tissue is damaged to only a small extent when inserting the medical needle.

The stylet illustrated in FIGS. 8 and 9 has a configuration in which a slope formed by the front end section 22 of the stylet forms a cutting face 22b. The cutting face is a front end face that forms an angle theta of 45° or less with the axis of the stylet and the needle main body. When the stylet is inserted into the needle main body, the entire cutting face 22b of the stylet protrudes from the front end opening 12 of the needle main body, and a front end face 11b of the needle section 11 of the needle main body is formed to be perpendicular to the axis of the needle main body, and does not form a cutting face (see FIG. 9). Therefore, a possibility of human tissue being damaged by the tip of the needle can be suppressed when performing a medical treatment in which a catheter or a laser fiber is inserted after removing the stylet.

The stylet illustrated in FIG. 10 has a configuration in which the helical coil has a quadrangular cross-sectional shape. In the example illustrated in FIG. 10, the helical coil forms a dense helix so that a gap is not formed in the rod-like section 22.

### REFERENCE SIGNS LIST

- 1: Needle main body
- 11: Needle section
- 11a: Cutting face
- 11b: Front end face
- 12: Front end opening
- 13: Base end block
- 2: Stylet
- 21: Rod-like section
- 22: Front end section
- 22a: Slope
- 22b: Cutting face
- 23: Base end block
- 24: Core

## Claims

1. A stylet for a medical needle comprising:
a rod-like section; and
a base end block that is provided at a base end of the rod-like section, the base end block being engaged with a base end block of a needle main body for positioning,
the rod-like section being inserted into the needle main body from a base end of the needle main body to close a front end opening of the needle main body, the entirety or part of the rod-like section being a helical coil formed by helically winding a wire.

2. The stylet for a medical needle according to claim 1, further comprising a core that is provided inside the helical coil.

3. The stylet for a medical needle according to claim 1 or 2, wherein a front end of the rod-like section has a structure in which a massive front end section is integrally provided to a front end of the helical coil.

4. The stylet for a medical needle according to claim 3, wherein the massive front end section has a slope that is formed by diagonally cutting the front end section.

5. A medical needle comprising:
the stylet according to any one of claims 1 to 4; and
a needle main body that includes a needle section, and a base end block that is provided at a base end of the needle section and engaged with a base end block of the stylet.

6. A medical needle comprising:
the stylet according to claim 4; and
a needle main body that includes a needle section, and a base end block that is provided at a base end of the needle section and engaged with a base end block of the stylet,
the slope entirely forming a cutting face that protrudes from the front end opening of the needle main body, and a front end face of the needle main body not forming a cutting face.
